# EUROPEAN PATENT SPECIFICATION

(11) **EP 0 877 818 B1**
(45) Date of publication and mention of the grant of the patent: **25.02.2004**
(21) Application number: 96938082.3
(22) Date of filing: 05.11.1996
(51) Int. Cl.: C12Q 1/48, C12Q 1/42, C12N 9/12

(54) **PROCESS FOR ACTIVATING A KINASE**
VERFAHREN ZUR AKTIVIERUNG EINER KINASE
PROCEDE POUR ACTIVER UNE KINASE

(30) Priority: 15.12.1995 GB 9525702
(43) Date of publication of application: 18.11.1998
(60) Divisional application: 01105452.5
(73) Proprietor: Novartis AG, 4056 Basel (CH); Novartis Pharma GmbH, 1230 Wien (AT)
(72) Inventor: HEMMINGS, Brian, Arthur, CH-4126 Bettingen (CH); ANDJELKOVIC, Mirjana, CH-4054 Basel (CH); CRON-HOFMANN, Peter, CH-4055 Basel (CH)
(74) Representative: Gros, Florent
(86) International application number: PCT/EP1996/004811
(87) International publication number: WO 1997/022716

(56) References cited:
- EP-A- 0 551 200
- WO-A-91/19008
- WO-A-92/09891
- WO-A-97/22360
- PROCEEDINGS OF THE NATIONAL ACADEMY OF SCIENCES OF THE UNITED STATES OF AMERICA 93 (12). 1996. 5699-5704. ISSN: 0027-8424, XP002031029 ANDJELKOVIC M ET AL: "Activation and phosphorylation of a pleckstrin homology domain containing protein kinase ( RAC - PK -PKB) promoted by serum and protein phosphatase inhibitors."
- BIOCHEMICAL JOURNAL 313 (1). 1996. 119-124. ISSN: 0264-6021, XP000671256 PERSAUD S J ET AL: "The mitogen-activated protein kinase pathway in rat islets of Langerhans: Studies on the regulation of insulin secretion."
- DIABETES 44 (6). 1995. 682-688. ISSN: 0012-1797, XP000671260 CAREY J O ET AL: "Okadaic acid, vanadate, and phenylarsine oxide stimulate 2-deoxyglucose transport in insulin -resistant human skeletal muscle."
- PROCEEDINGS OF THE NATIONAL ACADEMY OF SCIENCES OF USA, vol. 88, 1991, WASHINGTON US, pages 4171-4175, XP002031048 JONES P.F. ET AL: "Molecular cloning and identification of a serine/threonine protein kinase of the second-messenger subfamily" cited in the application
- Abstract of TOXICON, 1994, 32(3):339-350 " Characterization of natural toxins with inhibitory activity again serine/threonine protein phosphatases " by Honkanen R.E. et al. Retrieved from Internet on 18 July 2002 at URL: http://research.bmn.com/medline/search/record?u- id=MDLN.94287437&rendertype=ful
- LI Y.-M. AND CASIDA J.E.: 'Cantharidin-binding protein: identification as protein phosphatase 2A' PROCEEDINGS OF THE NATIONAL ACADEMY OF USA vol. 89, December 1992, pages 11867 - 11870
- SHEPPECK J.E. ET AL: 'Inhibition of the Ser-Thr phosphatases PP1 and PP2A by naturally occurring toxins' BIOORGANIC & MEDICINAL CHEMISTRY vol. 5, no. 9, 1997, pages 1739 - 1750

## Description

The present invention relates to a method for producing an active form of a kinase involved in an insulin dependent signalling pathway, and to the use of the active kinase in screening techniques.

### Background of the Invention

Protein phosphorylation and dephosphorylation are fundamental processes for the regulation of cellular functions. Protein phosphorylation is prominently involved in signal transduction, where extracellular signals are propagated and amplified by a cascade of protein phosphorylation and dephosphorylation. Two of the best characterised signal transduction pathways involve the c-AMP-dependant protein kinase (PKA) and protein kinase C (PKC). Each pathway uses a different second messenger molecule to activate the protein kinase, which, in turn, phosphorylates specific target molecules.

A novel subfamily of serine/threonine kinases has been recently identified and cloned, termed herein the RAC kinases (RAC-PK; Jones, et al. (1991) Proc. Natl Acad. Sci. USA 88, 4171-4175; Jones, et al. (1991) Cell Regulation 2, 1001-1009), but also known as PKB or Akt. RAC kinases have been identified in two closely related isoforms, RACα and RACβ, which share 90% homology at the gene sequence. Mouse RACα (c-akt) is the cellular homologue of the viral oncogene v-akt, generated by fusion of the Gag protein from the AKT8 retrovirus to the N-terminus of murine c-akt. Human RACβ is found to be involved in approximately 10% of ovarian carcinomas, suggesting an involvement of RAC kinases in cell growth regulation.

Another kinase implicated in cell growth control is S6 kinase, known as p70^{S6K}. S6 kinase phosphorylates the 40S ribosomal protein S6, an event which upregulates protein synthesis and is believed to be required in order for progression through the G₁ phase of the cell cycle. The activity of p70^{S6K} is regulated by serine/threonine phosphorylation thereof, and it is itself a serine/threonine kinase. The p70^{S6K} signalling pathway is believed to consist of a series of serine/threonine kinases, activating each other in turn and leading to a variety of effects associated with cell proliferation and growth. RAC-PK is believed to lie on the same signalling pathway as p70^{S6K}, but upstream thereof.

RAC-PK plays a major role in insulin-dependent signal transduction, which is important in a number of functions including the regulation of cell growth and glycogen metabolism. For example, glycogen synthase kinase-3 (GSK3), which is responsible for the activation of glycogen synthase, is a target for RAC-PK.

When isolated from natural sources, especially convenient sources such as tissue culture cells, RAC-PK and other signalling kinases are normally in the inactive state. In order to isolate active kinase proteins, it is necessary to stimulate cells in order to switch on the signalling pathway to yield active kinase. Moreover, when cells expressing kinase enzymes are used in kinase activity assays, it is necessary to employ activating agents prior to conducting the assay. Thus, cells are normally stimulated with mitogens and/or activating agents, such as IL-2, platelet-derived growth factor (PDGF), insulin, epidermal growth factor (EGF) and basic fibroblast growth factor (bFGF). Such agents are expensive and, when it is desired to produce active kinases or to activate cells in large amounts, the use of such agents is disadvantageous.

Screening of candidate compounds for activity as inhibitors of RAC-PK, or other signalling kinases in order to identify candidate immunosuppressive or antiproliferative agents requires a plentiful supply of kinase protein. Using modern day technology, it is possible to produce large quantities of virtually any desired protein in recombinant DNA expression systems. In the case of kinases such as those with which we are presently concerned, however, such systems are unsatisfactory because the proteins produced would be unphosphorylated and therefore inactive. There is therefore a requirement to identify a cost-effective way to produce phosphorylated kinase proteins which can be employed in screening procedures.

Okadaic acid, vanadate, and phenylarsine oxide have been found to stimulate 2-deoxyglucose transport in insulin-resistant human skeletal muscle (Carey et al., (1995) Diabetes 44, 682-688).

It is known (Janö *et al.,* (1988) Biochemistry, 85, 406-410) that vanadate can activate p70^{S6K} itself. The mechanism of this activation, however, is not known. We have now found that vanadate acts generally on signalling kinases, activating them and preventing deactivation by phosphatases. Moreover, we have found that okadaic acid, a different class of compound from vanadate which interacts with different proteins, may be used to similar effect.

### Summary of the Invention

According to the present invention, there is provided a process for activating a kinase of a signalling pathway comprising treatment thereof with a phosphatase inhibitor. Moreover, the invention provides methods for screening candidate immunosuppressive and antiproliferative agents using thus activated kinases.

### Detailed Description of the Invention

We have observed that modulation of RAC-PK activity appears to be effected by reversible phosphorylation, in which the equilibrium of the phosphorylation/dephosphorylation reaction is shifted in order to change the levels of active RAC-PK with respect to its inactive form. Build up of the active form may therefore be promoted by inhibition of the dephosphorylation reaction, achieved by treatment with a phosphatase inhibitor.

A surprising aspect of the present invention is that tyrosine phosphatase inhibitors, such as vanadate, are able to activate RAC-PK notwithstanding the fact that, as is disclosed herein, this kinase is activated by phosphorylation at threonine and serine residues.

It is known that vanadate activates p70^{S6K}. The invention accordingly does not extend to the use of vanadate to activate p70^{S6K}. However, the use of phosphatase inhibitors such as okadaic acid, which acts through a quite different mechanism, is part of the present invention.

As referred to herein, the signalling pathways are the activation cascades which ultimately regulate-signal transduction and kinases of these pathways are kinases whose in-vivo targets include at least one entity which contributes to such signal transduction. Preferably, the signalling pathways of the invention are insulin-dependent signalling pathways, which are responsible for transduction of signals from insulin and other growth factors. Without in any way wishing to place any limitation on the present invention, one such a pathway is believed to be triggered *in vivo* by binding of growth factors such as insulin and the like to their receptors, which stimulates *inter alia* phosphatidytinosito)-3-OH kinase (Pl-3K). Pl-3K in turn directly or indirectly phosphorylates RAC-PK, which indirectly leads to the eventual phosphorylation of p70^{S6K}.

Treatment of kinases according to the invention in order to activate them requires the exposure of the kinase to a phosphorylating agent, such as another kinase of the signalling pathway, and the phosphatase inhibitor. This may be accomplished, for example, *in vitro* by
(a) incubating together a kinase of a signalling pathway, an agent capable of phosphorylating the kinase in order to activate it, and a phosphatase inhibitor; and
(b) purifying the kinase from the incubation mixture.

The phosphorylating agent should be effective to phosphorylate the kinase on residues which lead to activation thereof. In the case of RAC-PK, the phosphorylating agent advantageously targets serine and threonine residues.

Preferably, the phosphorylating agent is one or more kinases of the signalling pathway which act, in the presence of suitable activating factors, to phosphorylate and thereby activate the kinase of interest. Preferably, this is accomplished by recovering active kinase enzyme form phosphatase-inhibitor treated cells, which contain the required signalling pathway kinases.

In the context of the present invention, *in vitro* signifies that the experiment is conducted outside a living organism or cell. *In vivo* includes cell culture. Treatment of cells *in vivo* with phosphatase inhibitors is especially effective for the preparation of active RAC-PK. However, since RAC-PK and other signaling kinases, for example p70^{S6K}, are on the same pathway, activation of RAC-PK results in the activation of other kinases on the same signalling pathway, for example p70^{S6K} itself. The invention therefore includes a method for activating kinases on signalling pathways in general, except for p70^{S6K}, especially where such kinases are downstream of RAC-PK in the pathway.

Cells which produce kinases which may be used in the present invention generally include any cell line of mammalian origin, especially fibroblast cell lines such as RAT-1, COS or NIH 3T3. Swiss 3T3 cells are particularly preferred. Where human cell lines are used, human embryonic kidney 293 cells are preferred.

Phosphatase inhibitors are agents which inhibit protein dephosphorylation by inhibiting the activity of phosphatase enzymes. A phosphatase has essentially the inverse activity of a kinase, and removes phosphate groups.

Examples of phosphatase inhibitors are vanadate and okadaic acid, with vanadate being the more effective agent in the case of RAC-PK. However, the action of vanadate is believed to be indirect, since it is a specific tyrosine phosphatase inhibitor and RAC-PK does not appear to be stimulated by tyrosine phosphorylation. Okadaic acid, on the other hand, which is known to act directly on phosphatase PP2A, appears to directly inhibit dephosphorylation of RAC-PK.

The use of other phosphatase inhibitors is envisaged and limited only by the suitability of such inhibitors for administration to the particular cell line being used. Vanadate and okadaic acid are believed to be generally applicable, but those of skill in the art will recognise that other phosphatase inhibitors are available and that their activity and suitability may easily be determined by routine empirical testing. For example, phosphatase inhibitors which may be suitable in the present invention include calyculin A, cantharidic acid, cantharidin, DTX-1, microcystin, nodularin and tautomycin. These and other phosphatase inhibitors are available commercially, e.g. from Calbiochem.

The phosphatase inhibitor is administered to cells in their normal growth medium, which may be serum free. Serum is itself observed to stimulate kinase activity, but is expensive and its function may be substituted by a phosphatase inhibitor according to the present invention. Suitable concentrations of phosphatase inhibitors include levels from 0.01 mM to 10mM, preferably 0.1 mM to 1mM. The most preferred concentration for vanadate is 0.1 mM.

The method of the invention may comprise additional steps intended to isolate the desired active kinase from the cells in which it is produced. Such steps are conventional procedures familiar to those skilled in the art and may be substituted for equivalent processes within the scope of the invention. The preferred process, however, comprises the steps of homogenising the cells, removing cell debris (for example by centrifugation) and separating the desired kinase by affinity purification.

Homogenisation may be carried out in a standard isotonic lysis buffer, advantageously containing a proteinase inhibitor such as phenylmethyl sulphonyl fluoride (PMSF) and a phosphatase inhibitor in order to inhibit deactivation of the kinase during the purification procedure. The cells are disrupted, thereby releasing the cytoplasmic and nuclear contents thereof into the lysis buffer.

Cell debris is then advantageously removed from the lysed cellular preparation, preferably by centrifuging the mixture in order to pellet all particulate matter. Only the soluble fraction remains in the supernatant.

The supernatant can then be subjected to standard protein purification techniques in order to isolate the kinase of interest if desired. Preferred methods, especially for relatively low volume preparations, involve affinity chromatography. Such techniques may employ an anti-kinase antibody or antiserum immobilised to a suitable matrix. Other immobilised binding agents, such as substrate analogues, may be employed.

Antibodies useful for immunoseparation of activated kinases according to the invention may be prepared according to techniques known in the art. In order to prepare a polyclonal serum, for example, an antigenic portion of the desired kinase, consisting of a peptide derived therefrom, such as a C-terminal peptide, or even the whole kinase, optionally in the presence of an adjuvant or conjugated to an immunostimulatory agent such as keyhole limpet haemocyanin, is injected into a mammal such as a mouse or a rabbit and antibodies are recovered therefrom by affinity purification using a solid-phase bound kinase or antigenic portion thereof. Monoclonal antibodies may be prepared according to established procedures.

Alternatively, and especially for larger scale preparations, separation procedures not involving affinity chromatography may be used.

For example, numerous methods are available in the art for separating polypeptides on the basis of size, such as chromatography and gel electrophoresis. Preferred are methods which perform a purification function as well as a size separating function, whilst not introducing unacceptable contaminants. Thus, methods such as step or continuous gradient centrifugation, particularly using sucrose gradients, dialysis techniques using controlled-pore membranes and membrane (Amicon) centrifugation are preferred. Especially preferred, however, is size exclusion chromatography, typically performed using porous beads as the chromatographic support. Size exclusion chromatography is, for example, described by Stellwagen, in Deutscher (1990) Guide to Protein Purification, Academic Press, Inc., San Diego, CA, USA, 317-328.

Alternative purification methods, described in general in Deutscher (1990), include chromatography based on separation by charge difference, such as ion exchange chromatography using an exchange group such as DEAE or CM bound to a solid phase packing material such as cellulose, dextran, agarose or polystyrene. Other methods include hydroxyapatite column chromatography (see, for example, Gorbunoff, (1985) Methods in Enzymology, 117, 370-380), and general affinity chromatography using glass beads or reactive dyes as affinity agents.

Advantageously, cation exchange chromatography may be employed, such that protein elution can be tailored to take into account the known or estimated pl of the kinase in question. The pl for any kinase may be determined experimentally, by isoelectric focusing. In this manner, it is possible selectively to elute from the cation exchange resin those proteins having a pl at or around that of the kinase, which results in a high degree of purification.

The invention further provides the use of an active kinase prepared according to the invention in a method for screening potential modulators of signalling pathways. Thus, the claimed method may comprise the additional step of exposing the kinase to a potential modulator and subsequently assessing the activity of the kinase in order to determine the effectiveness of the modulator.

The invention accordingly provides a method for screening candidate modulators of signalling pathways comprising:
(a) incubating together a kinase of a signalling pathway and a phosphatase inhibitor;
(b) adding a candidate modulator of the signalling pathway; and
(b) determining the activity of the kinase.

The exposure to the modulator may be performed on the activated or inactivated kinase either in a cell-free environment, optionally after purification of the kinase from the crude cellular preparation, or in situ in the cells which produce the kinase, after phosphatase inhibitor activation. Steps (a) and (b) may therefore be reversed, or conducted contemporaneously.

In step (a), especially if the assay is to be performed *in vitro,* an agent capable of phosphorylating the kinase may be added to the incubation mixture. Phosphatase inhibitors activate kinases by preventing dephosphorylation, so a phosphorylating agent will be required. Advantageously, the phosphorylating agent is a kinase of an insulin-dependent signalling pathway or an analogue thereof. Moreover, factors may be required to initiate or assist signal transduction in the signalling pathway. For example, it the compound being tested is a rapamycin analogue which binds FKBP, FKBP will be required in the incubation mixture.

Preferably, however, the procedure is carried out *in vivo* in cells containing kinases of the signalling pathway. In such an assay, the phosphatase inhibitor replaces serum or other agents previously employed as external stimulating agents to activate kinases of the signalling pathway.

The activity of the kinase may be assessed by means of a kinase activity assay, employing a substrate for the kinase. For example, myelin basic protein may be used, in accordance with established assay procedures. Physiological substrates, such as the 40S ribosomal subunit, or S6, may also be used. Alternatively, kinase activity may be assessed by determining the degree of phosphorylation of the kinase. Advantageously, phosphorylation on residues normally implicated in kinase activation is assessed. The identification of such residues, which is part of the present invention, is set forth below.

The assay of the invention may be used to measure the direct effect of the candidate compound on the assayed kinase, or it may be used to determine the effect of the compound on a kinase acting upstream thereof in the signalling pathway. In the latter situation, the assayed kinase acts as a substrate for the upstream kinase and the activity of the upstream kinase is assessed by determining the phosphorylation state or the activity of the assayed kinase.

In order to obtain a meaningful result, the activity of the assayed kinase exposed to the candidate modulator of the signalling pathway should be compared to the activity of the kinase not exposed to the agent, an inhibition of kinase activity being indicative of potential as an immunosuppressive or antiproliferative.

Compounds which demonstrate elevated levels of kinase inhibition may then be further assessed by determining the immunosuppressive or antiproliferative properties thereof directly, for instance by means of a cell proliferation inhibition assay. Such an assay preferably involves physical determination of T-cell proliferation in cells which have been subjected to kinase activation by a phosphatase inhibitor, exposed to the candidate kinase inhibitor and optionally subsequently stimulated with a mitogen, such as a growth factor, IL-2 or PMA. More simply, the assay may involve exposure of unstimulated cells to the candidate inhibitor, followed by stimulation with a phosphatase inhibitor.

According to a further aspect of the invention, we have been able to determine which sites are important for the phosphorylation of kinases, particularly those of the p70^{S6K}/RAC-PK family. Surprisingly, the majority of activating phosphorylation appears to take place on serine and threonine residues.

For example, an important activating residue is T308, present in the so-called T-loop between subdomains 7 and 8 of the kinase. A general guide to kinase structure is given in Woodgett (1994), *Protein Kinases,* IRL Press, UK. Another important activating residue is S473. Phosphorylation of this residue is required for full activation of RAC-PK *in vivo.*

The invention is further described, for the purposes of illustration only, in the following examples.

### Example 1

### Mitogenic stimulation and phosphorylation of RAC-PK.

The Swiss 3T3 cell line ( 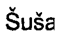 , M. & Thomas, G. (1990) *Proc. Natl. Acad. Sci. USA* **87**, 7040-7044) is utilised to investigate the possible involvement of RAC-PK in growth factor signalling. Quiescent Swiss 3T3 cells are serum-starved for 24 hr, followed by stimulation with 10% FCS.
(a) Kinase activity is assessed by immunoprecipitating RAC-PK and assaying the kinase using myelin basic protein as a substrate. Briefly, cell free extracts are prepared by scraping preconfluent cells into ice-cold TBS, lysing the cells in a buffer containing 50 mM Tris-HCl, pH 7.5, 1 mM EDTA, 1.0% Triton X-100, 2 mM EGTA, 1 mM PMSF, 20 µM leupeptin, 20 µM aprotenin and 10 µM molybdate. Lysates are centrifuged for 15 min at 12,000 x g at 4°C. RAC-PKa is immunoprecipitated from pasorbin-cleared extracts using a rabbit polyclonal antibody specific for the conserved C-terminus (anti-RAC⁴⁶⁹⁻⁴⁸⁰; Jones *et al.* (1991) *Proc. Natl. Acad. Sci. USA* **88**, 4171-4175) raised by injecting rabbits subcutaneously with the peptide FPQFSYSASSTA coupled to keyhole limpet haemocyanin and purified by precipitation using 50% (NH₄)₂SO₄ followed by affinity chromatography on RAC-PK coupled Affigel® 10 column (Bio-Rad). These antisera also recognise the b/AKT2 isoform, because its C-terminus differs from that of RAC-PKa in the last three amino acids. RAC-PK activity is assayed as described previously using myelin basic protein as substrate (Jones *et al.* (1991) *Proc. Natl. Acad. Sci. USA* **88**, 4171-4175). The extracts are incubated for 2hrs at 4°C with the antiserum (2µg/100µl extract) the immunoprecipitates collected using Protein A sepharose and washed with lysis buffer. The protein sepharose beads are resuspended in 100 µl of 10 mM Tris-HCl pH 7.5, 1 mM DTT, 10µm molybdate and 35µl used for the kinase assay, as follows:
   Reaction mixtures in a final volume of 50 µl contain 50 mM Tris-HCI, pH 7.5, 10 mM MgCl₂, 1 mM DTT, 1 mM protein kinase inhibitor, PKI peptide, 25 µg of myelin basic protein (MBP-Sigma), 50 µM (γ-³²P) ATP (3500 cpm/pmol) and 35 µl of immunoprecipitate from cell free extracts or purified fractions of RAC protein kinase. After incubation at 30°C for 10, 30 or 60 minutes samples are analysed by 12% SDS/PAGE followed by autoradiography and quantified by scintillation counting of the phosphorylated MBP bands.
   Immunoprecipitated RAC-PK activity is found to be 2 to 4-fold higher in serum-stimulated cells versus quiescent cells. Activation occurs within 5 min and kinase activity remains elevated for at least 120 min.
(b) Activation coincides with decreased mobility of RAC-PK on SDS-PAGE. In order to determine which forms are present on SDS-PAGE gels, immunoblotting is performed using anti-RAC-PK antisera prepared as above. Cell extracts and immunoprecipitates are resolved by 7.5% SDS-PAGE, transferred to Immobilon-P membranes (Millipore) and incubated with the anti-RAC⁴⁶⁹⁻⁴⁸⁰ antibody. Detection is performed using alkaline phosphatase-conjugated anti-rabbit antibody.
   At least three different forms can be detected by immunoblot analysis, termed **a**, **b** and **c**. The kinase from quiescent cells migrates as a doublet of the **a** and **b** forms and during stimulation a slower migrating form c appears, followed by disappearance of form a. These results suggest that RAC-PK activity is modulated by reversible phosphorylation.
(c) To test this possibility the *in vivo* effects of phosphatase inhibitors okadaic acid and vanadate on RAC-PK from Swiss 3T3 cells are examined. Cells are serum-starved for 24 hrs, followed by stimulation with 1 µM okadaic acid, or 0.1 mM vanadate prepared with 0.1 mM H₂O₂ (Posner, *et al.* (1994) *J. Biol. Chem.* **269**, 4596-4604), optionally in conjunction with 10% FCS. Treatment of cells with okadaic acid, a specific inhibitor of PP2A and PP1, induces a 3-fold increase in RAC-PK activity and decreases electrophoretic mobility. Simultaneous treatment with 1 µM okadaic acid and 10% serum causes a 5-fold activation and a larger alteration of the electrophoretic mobility. An 11-fold activation is observed following-treatment with 0.1 mM vanadate, which converts the major part of the protein into the slowest-migrating form **c**.
   In order to confirm that multiple electrophoretic mobility forms reflect different phosphorylation states of the kinase, RAC-PK is immunoprecipitated from ³²P-labeled quiescent and vanadate-stimulated Swiss 3T3 cells. Swiss 3T3 cells are arrested in phosphate-free DMEM/FCS as described ( 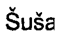 , M. & Thomas, G. (1990) *Proc. Natl. Acad. Sci. USA* **87**, 7040-7044) and serum-starved for 16 hr prior to labelling with [³²P]orthophosphate for 6 - 10 hr (2 mCi per 15 cm dish). Stimulation is performed 0.1 mM vanadate. Quantification of phosphorylation is performed using the ImageQuant software. Vanadate treatment leads to a 3 to 4-fold increase in phosphorylation, demonstrating that the mobility forms b and c represent phosphorylated RAC-PK.
(d) In order to determine which residues are phosphorylated in activated RAC-PK, phosphoamino acid analysis is carried out on cells labelled as above according to Boyle, et *al.* (1991) *Methods Enzymol.* **201**, 110-149. The kinase from arrested cells appears phosphorylated mainly on serine residues, and at low levels on threonine, with a ratio of 12:1. Vanadate stimulation leads to an increase in phosphoserine and in particular in phosphothreonine content, reducing the ratio to 4:1. Phosphotyrosine is not detected after vanadate stimulation, either by phosphoamino acid analysis, or by immunoblot analysis using an anti-phosphotyrosine antibody. These results show that RAC-PK is activated by a phosphorylation mechanism. Furthermore, we conclude that RAC-PK activation mediated by vanadate is probably indirect, since vanadate is known to be an inhibitor of tyrosine phosphatases.

### Example 2

### Inactivation of RAC-PK by Protein Phosphatase 2A in Vitro.

To confirm that RAC-PK is regulated by phosphorylation the effects of Protein Phosphatase 2A (PP2A) treatment on the kinase immunoprecipitated from quiescent and vanadate-stimulated Swiss 3T3 cells are investigated. As treatment of cells with 1 mM okadaic acid for 2 hr preferentially inactivates PP2A rather than PP1, RAC-PK is incubated either with the purified PP2A catalytic subunit (PP2Ac), or PP2A dimer consisting of the catalytic and regulatory PR65 subunit (PP2A₂).

Immunoprecipitated RAC-PK is incubated with 0.3 U/ml of porcine muscle PP2Ac or 1.7 U/ml of rabbit muscle PP2A₂ in 30 ml buffer containing 50 mM Tris-HCl 7.5,1% b-mercaptoethanol, 1 mM MnCl₂, 1 mM benzamidine and 0.5 mM phenylmethylsulfonyl fluoride at 30°C for 60 min (one unit (U) is defined as one nmol of Pi released from phosphorylase a per min). The reactions are stopped by addition of 50 nM calyculin A. The immune complexes formed are washed with 50 mM Tris-HCl pH 7.5, 1 mM benzamidine, 0.5 mM phenylmethylsulfonyl fluoride and 50 nM calyculin A and RAC-PK is assayed as described above.

Dephosphorylation of the activated RAC-PK *in vitro* by PP2Ac results in an 84 % reduction of kinase activity and concomitant change in electrophoretic mobility, converting it from form **c** to **b**. PP2A₂ treatment leads to a 92 % reduction of activity and restores the protein mobility on SDS-PAGE to the **a/b** doublet. These results confirm that the activity changes observed are achieved by a reversible phosphorylation mechanism. Moreover, PP2A is indicated as a potential regulator of RAC-PK activity *in vivo.*

### Example 3

### RAC-PKa Stimulates p70^{s6k} Activity.

In Swiss 3T3 cells RAC-PK is activated by insulin (4.5-fold), comparable to levels detected for p70^{s6k}. In contrast, insulin has little or no effect on p42^{mapk} and p44^{mapk} in these cells, suggesting that RAC-PK and p70^{s6k} may reside on the same signalling pathway, which is a different pathway to the MAPK pathway.

In order to investigate this possibility the effects of wortmannin and rapamycin on serum induced activation of the two kinases is examined. Wortmannin, an inhibitor of phosphoinositide (Pl) 3-kinase, and immunosuppressant rapamycin block the activation of p70^{s6k} by affecting the same set of phosphorylation sites.

Stimulation of quiescent Swiss 3T3 fibroblasts leads to a ~4-fold induction of RAC-PK activity, whereas wortmannin treatment preceding serum stimulation almost completely blocks the activation. On the other hand, rapamycin pretreatment does not exert any significant effect on RAC-PK activation. Wortmannin also blocks the appearance of slowest RAC-PK mobility form that is observed following serum treatment, while rapamycin does not affect RAC-PK mobility. In the same experiment wortmannin and rapamycin pretreatment abolish p70^{s6k} activation.

These results suggest that RAC-PK may lie upstream of p70^{S6K} on the p70^{S6K} signalling pathway, which is inhibited upstream of RAC-PK by wortmannin and downstream thereof by rapamycin. To examine this possibility, the regulation of p70^{s6k} is investigated in a transient cotransfection assay using human 293 cells. RAC-PKα constructs are prepared by ligating the RAC-PKa cDNA (Jones *et al.* (1991) *Proc. Natl. Acad. Sci. USA* **88**, 4171-4175) in-frame to the initiator methionine, in the mammalian expression vector pECE. The construct is also subcloned into a CMV promoter-driven expression vector. The construct is confirmed by restriction analysis and sequencing. Constructs expressing Myc-tagged p70^{s6k} are obtained from Dr. G. Thomas (Friederich Miescher Institut, Basel, Switzerland). Constructs are transfected into COS cells using standard procedures. Coexpression of RAC-PKa with p70^{s6k}-Myc results in a 3.5- and 3-fold increase of basal and insulin-stimulated p70^{s6k}-Myc activity, respectively.

## Claims

1. A process for producing active RAC-PK by specifically shifting the equilibrium of a phosphorylation/dephosphorylation reaction directed to at least one serine and/or threonine residue of said kinase towards phosphorylation, comprising treatment of said RAC-PK with vanadate or an inhibitor of phosphatase PP2A.

2. A process according to claim 1 wherein active RAC-PK is isolated.

3. A process according to claim 1 or 2 which is carried out in cell culture which contain at least one kinase upstream of RAC-PK in its signalling pathway which is capable of phosphorylating RAC-PK on at least one serine and/or threonine residue.

4. A process according to claim 1 or 2 which is carried out *in* vitro, comprising:
(a) incubating together RAC-PK, an agent capable of phosphorylating RAC-PK on at least one serine and/or threonine residue, and vanadate or an inhibitor of phosphatase PP2A ; and
(b) purifying active RAC-PK from the incubation mixture.

5. A process according to claim 4, wherein the phosphorylating agent is at least one kinase upstream of RAC-PK in its signalling pathway which is capable of phosphorylating RAC-PK on at least one serine and/or threonine residue, thereby activating it.

6. A process according to claim 4 or 5, wherein said serine residue is S473.

7. A process according to claim 4 or 5, wherein said threonine residue is T308.

8. A process for screening candidate compounds for activity as inhibitors of RAC-PKin cell culture, comprising:
(a) incubating together cells which contain RAC-PK and a vanadate or an inhibitor of phosphatase PP2A;
(b) adding the candidate compound; and
(c) determining the activity of RAC-PK.

9. A process for screening candidate compounds for activity as inhibitors of RAC-PK *in vitro,* comprising:
(a) incubating together RAC-PK, an agent capable of phosphorylating RAC-PK on at least one serine and/or threonine residue, and vanadate or an inhibitor of phosphatase PP2A ;
(b) adding the candidate compound; and
(c) determining the activity of RAC-PK.

10. A process according to claim 9, wherein the phosphorylating agent is one or more kinases upstream of RAC-PK.

11. A process for screening candidate compounds for activity as inhibitors of RAC-PK, comprising:
(a) incubating active RAC-PK obtainable by a process according to any of claims 1 to 7, together with vanadate or an inhibitor of phosphatase PP2A ;
(b) adding the candidate compound; and
(c) determining the activity of RAC-PK.

12. A process for screening potential modulators of RAC-PK which directly or indirectly effect activation of RAC-PK, comprising:
(a) incubating RAC-PK according to claim 9(a) or according to claim 11(a), with an agent capable of phosphorylating RAC-PK on at least one serine and/or threonine residue, and, vanadate or an inhibitor of phosphatase PP2A ;
(b) adding the potential modulator of RAC-PK; and
(c) assessing the activity of the modulator.

13. A process according to claim 12, wherein the potential modulator of RAC-PK is a kinase upstream of RAC-PK.

14. A process according to claim 9 or 12, wherein said serine residue is S473.

15. A process according to claim 9 or 12, wherein said threonine residue is T308.

16. A process according to claims 8, 9, 11, or 12, wherein steps (a) and (b) are performed contemporaneously.

17. A process according to any of the preceding claims, wherein the inhibitor of phosphatase PPA2 is selected from the group consisting of okadaic acid and calyculin A.

## Patentansprüche

1. Verfahren zur Herstellung eines aktiven RAC-PK durch spezifisches Verschieben des Gleichgewichts einer Phosphorylierungs/Dephosphorylierungsreaktion, die auf mindestens einen Serin- und/oder Threoninrest der Kinase gerichtet ist, in Richtung der Phosphorylierung, mit einer Behandlung des RAC-PK mit Vanadat oder einem Inhibitor der Phosphatase PP2A.

2. Verfahren nach Anspruch 1, bei dem das aktive RAC-PK isoliert wird.

3. Verfahren nach Anspruch 1 oder 2, das in Zellkultur ausgeführt wird, die mindestens eine Kinase stromaufwärts vom RAC-PK in seinem Signalweg enthält, die fähig ist, RAC-PK an mindestens einem Serin- und/oder Threoninrest zu phosphorylieren.

4. Verfahren nach Anspruch 1 oder 2, das in vitro ausgeführt wird, mit
(a) zusammen Inkubieren von RAC-PK, einem Mittel, das fähig ist, RAC-PK an mindestens einem Serin- und/oder Threoninrest zu phosphorylieren, einem Vanadat oder einem Inhibitor der Phosphatase PP2A, und
(b) Reinigen des aktiven RAC-PK aus der Inkubationsmischung.

5. Verfahren nach Anspruch 4, bei dem das Phosphorylierungsmittel mindestens eine Kinase stromaufwärts des RAC-PK in seinem Signalweg ist, die fähig ist, RAC-PK an mindestens einem Serin- und/oder Threoninrest zu phosphorylieren, wodurch es aktiviert wird.

6. Verfahren nach Anspruch 4 oder 5, bei dem der Serinrest S473 ist.

7. Verfahren nach Anspruch 4 oder 5, bei dem der Threoninrest T308 ist.

8. Verfahren zum Screenen möglicher Verbindungen auf eine Aktivität als Inhibitoren von RAC-PK in Zellkultur, mit:
(a) zusammen Inkubieren von Zellen, die RAC-PK enthalten, einem Vanadat oder einem Inhibitor der Phosphatase PP2A,
(b) Zugeben der möglichen Verbindung, und
(c) Bestimmen der Aktivität von RAC-PK.

9. Verfahren zum Screenen möglicher Verbindungen auf eine Aktivität als Inhibitoren von RAC-PK in vitro, mit:
(a) zusammen Inkubieren von RAC-PK, einem Mittel, das fähig ist, RAC-PK an mindestens einem Serin- und/oder Threoninrest zu phosphorylieren, einem Vanadat oder einem Inhibitor der Phosphatase PP2A,
(b) Zugeben der möglichen Verbindung, und
(c) Bestimmen der Aktivität von RAC-PK.

10. Verfahren nach Anspruch 9, bei dem das Phosphorylierungsmittel eine oder mehrere Kinasen stromaufwärts vom RAC-PK ist.

11. Verfahren zum Screenen möglicher Verbindungen auf eine Aktivität als Inhibitoren von RAC-PK, mit:
(a) Inkubieren des aktiven RAC-PK's, das durch ein Verfahren nach einem der Ansprüche 1 bis 7 erhältlich ist, zusammen mit Vanadat oder einem Inhibitor der Phosphatase PP2A,
(b) Zugeben der möglichen Verbindung, und
(c) Bestimmen der Wirksamkeit von RAC-PK.

12. Verfahren zum Screenen potentieller Modulatoren von RAC-PK, die direkt oder indirekt die Aktivierung von RAC-PK bewirken, mit:
(a) Inkubieren von RAC-PK gemäß Anspruch 9(a) oder gemäß Anspruch 11(a) mit einem Mittel, das fähig ist, RAC-PK an mindestens einem Serin- und/oder Threoninrest zu phosphorylieren, und Vanadat oder einem Inhibitor der Phosphatase PP2A,
(b) Zugeben des potentiellen Modulators von RAK-PK, und
(c) Bewerten der Aktivität des Modulators.

13. Verfahren nach Anspruch 12, bei dem der potentielle Modulator des RAC-PK eine Kinase stromaufwärts vom RAC-PK ist.

14. Verfahren nach Anspruch 9 oder 12, bei dem der Serinrest S473 ist.

15. Verfahren nach Anspruch 9 oder 12, bei dem der Threoninrest T308 ist.

16. Verfahren nach den Ansprüchen 8, 9, 11 oder 12, bei dem die Schritte (a) und (b) gleichzeitig ausgeführt werden.

17. Verfahren nach einem der vorstehenden Ansprüche, bei dem der Inhibitor der Phosphatase PP2A aus der Gruppe ausgewählt ist, die aus Okadaic Acid und Calyculin A besteht.

## Revendications

1. Procédé de préparation de RAC-PK active en déplaçant spécifiquement l'équilibre d'une réaction de phosphorylation/déphosphorylation dirigée vers au moins un résidu sérine et/ou thréonine de ladite kinase vers la phosphorylation, comprenant le traitement de ladite RAC-PK avec du vanadate ou un inhibiteur de phosphatase PP2A.

2. Procédé suivant la revendication 1, dans lequel on isole la RAC-PK active.

3. Procédé suivant la revendication 1 ou 2, conduit dans une culture cellulaire qui contient au moins une kinase en amont de la RAC-PK dans son trajet de signalement, qui est capable de phosphoryler la RAC-PK sur au moins un résidu sérine et/ou thréonine.

4. Procédé suivant la revendication 1 ou 2, qui est effectué *in vitro,* comprenant :
(a) l'incubation conjointe de la RAC-PK, d'un agent capable de phosphoryler la RAC-PK sur au moins un résidu sérine et/ou thréonine, et de vanadate ou d'un inhibiteur de phosphatase PP2A ;
et
(b) la purification de la RAC-PK active à partir du mélange d'incubation.

5. Procédé suivant la revendication 4, dans lequel l'agent de phosphorylation est au moins une kinase en amont de la RAC-PK dans son trajet de signalement, qui est capable de phosphoryler la RAC-PK sur au moins un résidu sérine et/ou thréonine, activant ainsi celle-ci.

6. Procédé suivant la revendication 4 ou 5, dans lequel ledit résidu sérine est S473.

7. Procédé suivant la revendication 4 ou 5, dans lequel ledit résidu thréonine est T308.

8. Procédé de sélection de composés candidats pour leur activité en tant qu'inhibiteurs d'une culture cellulaire de RAC-PKin, comprenant :
(a) l'incubation conjointe des cellules qui contiennent la RAC-PK et de vanadate ou d'un inhibiteur de phosphatase PP2A ;
(b) l'addition du composé candidat ; et
(c) la détermination de l'activité de RAC-PK.

9. Procédé de sélection de composés candidats pour leur activité en tant qu'inhibiteurs de la RAC-PK *in vitro,* comprenant :
(a) l'incubation conjointe de la RAC-PK, d'un agent capable de phosphoryler la RAC-PK sur au moins un résidu sérine et/ou thréonine, et de vanadate ou d'un inhibiteur de phosphatase PP2A ;
(b) l'addition du composé candidat ; et
(c) la détermination de l'activité de RAC-PK.

10. Procédé suivant la revendication 9, dans lequel l'agent de phosphorylation est constitué d'une ou plusieurs kinases en amont de RAC-PK.

11. Procédé de sélection de composés candidats pour leur activité en tant qu'inhibiteurs de la RAC-PK, comprenant :
(a) l'incubation de la RAC-PK active susceptible d'être obtenue par un procédé suivant l'une quelconque des revendications 1 à 7, conjointement avec du vanadate ou un inhibiteur de phosphatase PP2A ;
(b) l'addition du composé candidat ; et
(c) la détermination de l'activité de RAC-PK.

12. Procédé de sélection de modulateurs potentiels de RAC-PK qui effectuent directement ou indirectement l'activation de RAC-PK, comprenant :
(a) l'incubation de la RAC-PK selon la revendication 9(a) ou selon la revendication 11(a), avec un agent capable de phosphoryler la RAC-PK sur au moins un résidu sérine et/ou thréonine, et du vanadate ou d'un inhibiteur de phosphatase PP2A ;
(b) l'addition du modulateur potentiel de RAC-PK ; et
(c) l'évacuation de l'activité du modulateur.

13. Procédé suivant la revendication 12, dans lequel le modulateur potentiel de la RAC-PK est une kinase en amont de RAC-PK.

14. Procédé suivant la revendication 9 ou 12, dans lequel ledit résidu sérine est S473.

15. Procédé suivant la revendication 9 ou 12, dans lequel ledit résidu thréonine est T308.

16. Procédé suivant les revendications 8, 9, 11 ou 12, dans lequel les étapes (a) et (b) sont effectuées en même temps.

17. Procédé suivant l'une quelconque des revendications précédentes, dans lequel l'inhibiteur de phosphatase PP2A est choisi dans le groupe constitué par l'acide okadaïque et la calyculine A.
